# EUROPEAN PATENT APPLICATION

(11) **EP 4 681 643 A1**
(43) Date of publication of application: **21.01.2026**
(21) Application number: 24770455.4
(22) Date of filing: 21.02.2024
(51) Int. Cl.: A61B 5/276, A61B 5/256, A61B 5/28, A61B 5/346

(54) **BIOLOGICAL SIGNAL MEASUREMENT DEVICE AND METHOD FOR CONTROLLING BIOLOGICAL SIGNAL MEASUREMENT DEVICE**

(30) Priority: 15.03.2023 JP 2023040872
(71) Applicant: Omron Corporation, Kyoto-shi, Kyoto 600-8530 (JP); Omron Healthcare Co., Ltd., Muko-shi, Kyoto 617-0002 (JP)
(72) Inventor: KIMURA ISHIDA, Yui, Kyoto-shi, Kyoto 600-8530 (JP); KOIZUMI, Masayuki, Kyoto-shi, Kyoto 600-8530 (JP); KUBO, Mitsuaki, Kyoto-shi, Kyoto 600-8530 (JP); WANG, Danni, Kyoto-shi, Kyoto 600-8530 (JP); KAWABATA, Yasuhiro, Muko-shi, Kyoto 617-0002 (JP); FUJII, Kenji, Muko-shi, Kyoto 617-0002 (JP); MATSUMURA, Naomi, Muko-shi, Kyoto 617-0002 (JP); FUKUNAGA, Seiji, Muko-shi, Kyoto 617-0002 (JP); YOSHIDA, Takuya, Kyoto-shi, Kyoto 600-8234 (JP)
(74) Representative: Isarpatent
(86) International application number: PCT/JP2024/006331
(87) International publication number: WO 2024/190342

(57) **Abstract**

A biological signal measurement device includes a dry-type electrode; a member configured to fix the electrode in a state in which the electrode is pressed against a living body; and a control body configured to measure a biological signal by the electrode. The control body includes a waveform feature extraction unit configured to extract a feature amount from a waveform of the biological signal, and an attachment state determination unit configured to determine whether an attachment state of the electrode is good or poor, based on a feature amount extracted from a waveform of a biological signal measured during a period after an elapsed time from attachment of the electrode to the living body by the member reaches a predetermined time.

## Description

### TECHNICAL FIELD

The present invention relates to a biological signal measurement device for measuring a biological signal by an electrode fixed to a surface of the skin.

### BACKGROUND ART

A technique for fixing an electrode to the surface of the skin of a living body and measuring a biological signal such as an electrocardiogram or electromyogram is known, and such a technique is applied to various measurement devices. In this type of device, if a contact state between the electrode and the surface of the skin is not appropriate, the measurement accuracy may decrease or the measurement itself may become impossible.

Patent Literature 1 proposes an idea of determining, before starting the recording of an electrocardiogram in the measurement of a standard 12-lead electrocardiogram, whether noise is mixed, whether an electrode is erroneously attached, and whether an electrode is detached, based on features of individual lead waveforms.

### CITATION LIST

### PATENT LITERATURE

Patent Literature 1: JP 2009-261723 A

### SUMMARY OF INVENTION

### TECHNICAL PROBLEM

The present inventors have been developing a type of measurement device in which an electrode is fixed to the surface of the skin by wrapping a band in which a dry-type electrode is embedded (hereinafter, also referred to as "electrode band") around an arm or a leg. By adopting such a fixing structure of the dry-type electrode, there are advantages in that the measurement can be easily performed because the patient himself/herself can easily attach and detach the device, and long-term measurement can be easily performed while wearing the device because there is no skin rash or the like. However, on the other hand, if the patient himself/herself is allowed to attach and detach the device, it is expected that the frequency of attachment failure will increase. Therefore, from the perspective of improving the reliability and stability of measurement, it is desirable to provide a mechanism that automatically determines the attachment state of the dry-type electrode and notifies the patient when attachment failure occurs.

The present inventors have experimentally fabricated electrode bands and conducted repeated attachment tests, and have found that there are two causes of attachment failure of the electrode, which are improper contact of the electrode with the surface of the skin (physical connection failure) and insufficient wetting between the electrode and the surface of the skin. Furthermore, it has been found that in many cases, insufficient wetting is resolved by perspiration after a certain period of time has elapsed since the electrode was attached, and a certain number of patients do not have sufficient wetting between the electrode and the skin even after a certain period of time has elapsed.

In the 12-lead electrocardiogram as in Patent Literature 1, a wet-type electrode using a conductive gel is usually used, and thus attachment failure due to insufficient wetting is not assumed. Therefore, when the determination method of Patent Literature 1 is simply applied to a dry-type electrode device, it is impossible to distinguish whether the cause is insufficient wetting or physical connection failure, and a determination result of attachment failure is uniformly output. However, when the cause is insufficient wetting, it is highly likely to be resolved over time, and therefore it is not appropriate to immediately determine an attachment failure. Rather, in the case of insufficient wetting, the patient is misguided because the cause is not resolved even when the electrode is re-attached.

The present invention has been made in view of the above situations, and an object thereof is to provide a technique for appropriately determining whether an attachment state of an electrode is good or poor, and for appropriately notifying a user of a determination result in a biological signal measurement device employing a dry-type electrode.

### SOLUTION TO PROBLEM

The present disclosure includes a biological signal measurement device including a dry-type electrode, a member configured to fix the electrode in a state in which the electrode is pressed against a living body, and a control body configured to measure a biological signal by the electrode, in which the control body includes a waveform feature extraction unit configured to extract a feature amount from a waveform of the biological signal, and an attachment state determination unit configured to determine whether an attachment state of the electrode is good or poor, based on a feature amount extracted from a waveform of a biological signal measured during a period after an elapsed time from attachment of the electrode to the living body by the member reaches a predetermined time.

The predetermined time may be set to a time of five minutes or more.

The waveform feature extraction unit may extract, as the feature amount, a length of a non-signal section in which a state in which the biological signal is saturated or 0 continues, and the attachment state determination unit may determine the attachment state to be poor when the non-signal section having a length equal to or greater than a predetermined value appears.

The attachment state determination unit may determine the attachment state to be poor before the elapsed time reaches the predetermined time, when a feature amount extracted from a waveform of a biological signal measured during a period before the elapsed time reaches the predetermined time satisfies a predetermined criterion.

The dry-type electrode may include a plurality of electrode pairs, the waveform feature extraction unit may extract, as the feature amount, a length of a non-signal section in which a state in which the biological signal is saturated or 0 continues, and the predetermined criterion may be a fact that the non-signal section having a length equal to or greater than a predetermined value appears in all of the plurality of electrode pairs in a first period, but the non-signal section having the length equal to or greater than the predetermined value does not appear in some of the plurality of electrode pairs in a second period after the first period.

The dry-type electrode may include a plurality of electrode pairs, the waveform feature extraction unit may extract, as the feature amount, a length of a non-signal section in which a state in which the biological signal is saturated or 0 continues, and the predetermined criterion may be a fact that the non-signal section having a length equal to or greater than a predetermined value appears in none of the plurality of electrode pairs in a first period, but the non-signal section having the length equal to or greater than the predetermined value appears in some of the plurality of electrode pairs in a second period after the first period.

The dry-type electrode may include a plurality of electrode pairs, the waveform feature extraction unit may extract, as the feature amount, a length of a non-signal section in which a state in which the biological signal is saturated or 0 continues, and the predetermined criterion may be a fact that an electrode pair in which the non-signal section having a length equal to or greater than a predetermined value appears and an electrode pair in which the non-signal section having the length equal to or greater than the predetermined value does not appear are mixed in the plurality of electrode pairs.

A notification unit configured to notify a user when the attachment state determination unit determines the attachment state to be poor may be further included.

The attachment state determination unit may determine a cause of a poor attachment state, based on a feature amount extracted from a waveform of the biological signal, and the notification unit may vary a notification to the user in accordance with the cause of the poor attachment state.

The waveform feature extraction unit may extract, as the feature amount, a length of a non-signal section in which a state in which the biological signal is saturated or 0 continues, and the notification unit may prompt the user to re-attach the electrode when the attachment state determination unit determines that the non-signal section having a length equal to or greater than a predetermined value appears.

The waveform feature extraction unit may extract, as the feature amount, an amplitude of a peak included in the biological signal, and the notification unit may prompt the user to change an attachment position of the electrode when the attachment state determination unit determines that the amplitude of the peak is smaller than a predetermined amplitude value.

The waveform feature extraction unit may extract, as the feature amount, a degree of noise contamination in the biological signal, and the notification unit may prompt the user to take a measure to increase wetting between the electrode and a surface of a skin when the attachment state determination unit determines that the degree of noise contamination is equal to or greater than a predetermined threshold value.

The control body may include an operation unit that is operated by a user after the user attaches the electrode, and may start counting the elapsed time when the user operates the operation unit.

The biological signal may be an electrocardiogram (ECG) signal.

The member may be a band provided with the electrode.

The band may be attached to an upper arm of the living body.

The present disclosure includes a method for controlling a biological signal measurement device configured to measure a biological signal by a dry-type electrode, the method including counting an elapsed time from attachment of the electrode to a living body, extracting a feature amount from a waveform of the biological signal, and determining whether an attachment state of the electrode is good or poor, based on a feature amount extracted from a waveform of a biological signal measured during a period after the elapsed time reaches a predetermined time.

The present invention may be regarded as a biological signal measurement device including at least a part of the above configuration, or may be regarded as an electrocardiogram measurement device that measures an ECG signal as a biological signal. Alternatively, the present invention may be regarded as an attachment state determination device that can be mounted on a biological signal measurement device. Further, the present invention can be regarded as an attachment state determination method including at least a part of the above processing, a method for controlling a biological signal measurement device, or a program for implementing such a method and a non-transitory recording medium on which the program is recorded. Note that the present invention can be configured by combining each of the above-described configurations and processes to the extent possible.

### ADVANTAGEOUS EFFECTS OF INVENTION

According to the present invention, it is possible to appropriately determine whether an attachment state of an electrode is good or poor, and to appropriately notify a user of a determination result in a biological signal measurement device employing a dry-type electrode.

### BRIEF DESCRIPTION OF THE DRAWINGS

[FIG. 1] FIG. 1 is a diagram illustrating a state in which a biological signal measurement device is worn on an upper arm.
[FIG. 2] FIG. 2 is a plan view of the biological signal measurement device.
[FIG. 3] FIG. 3 is a perspective view of the biological signal measurement device.
[FIG. 4] FIG. 4 is a block diagram illustrating a functional configuration of a control body.
[FIG. 5] FIG. 5 is a flowchart illustrating a flow of an ECG signal measurement process.
[FIG. 6] FIG. 6 is a diagram illustrating an example of an electrode pair and an ECG signal.
[FIG. 7] FIG. 7 is a diagram illustrating a waveform of an ECG signal and heart rate information.
[FIG. 8] FIG. 8 is a flowchart illustrating a flow of an attachment state determination process in a first embodiment.
[FIG. 9A] FIG. 9A is a diagram illustrating an example of an ECG signal waveform measured in a resting state.
[FIG. 9B] FIG. 9B is a diagram illustrating an example of an ECG signal waveform distorted due to a body movement.
[FIG. 9C] FIG. 9C is a diagram illustrating an example of an ECG signal waveform measured when there is an attachment failure of an electrode.
[FIG. 10] FIG. 10 is a flowchart illustrating a flow of an attachment state determination process in a second embodiment.
[FIG. 11] FIG. 11 is a flowchart illustrating a flow of an attachment state determination process in a third embodiment.
[FIG. 12] FIG. 12 is a diagram illustrating an example of a criterion used when determining an attachment state in the third embodiment.

### DESCRIPTION OF EMBODIMENTS

### <Application Example>

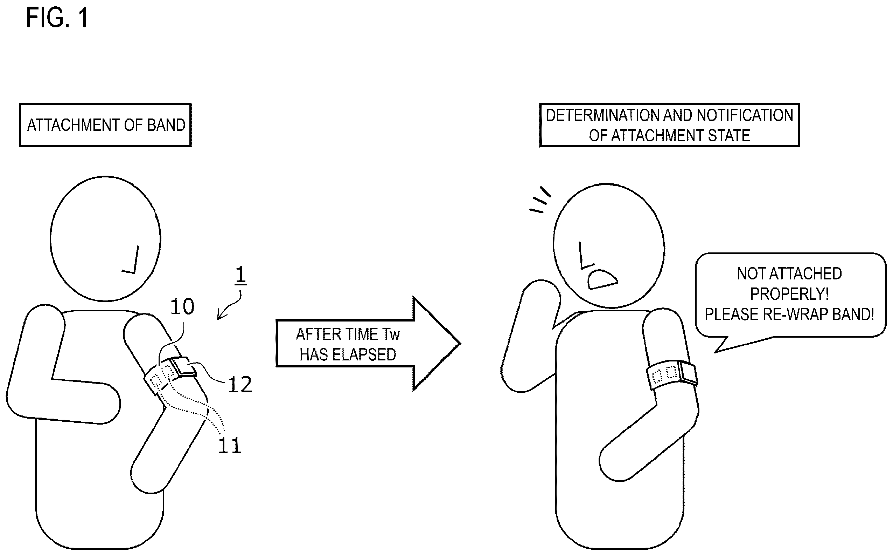

An application example of the present invention will be described with reference to FIG. 1.

A biological signal measurement device 1 is a portable measurement device that is used while being worn on a living body. The biological signal measurement device 1 includes, as main components, a band 10 provided with one or more dry-type electrodes 11, and a control body 12 that measures a biological signal by the electrodes 11.

The biological signal to be measured is also called a bioelectric signal or a biopotential, and is an electric signal generated in accordance with the activity of a living body. Examples include an electrocardiogram signal (a minute electrical signal associated with a heart rate), a myoelectric signal (a minute electrical signal associated with muscle activity), an electroencephalogram signal (a minute electrical signal associated with brain activity), and the like. Examples of measurement sites where the band 10 can be attached include upper limbs (upper arms, forearms, wrists, hands, and fingers), lower limbs (thighs, lower legs, ankles, feet, and toes), head, neck, chest, abdomen, and earlobe, and a measurement site is appropriately selected depending on the type of biological signal to be measured, a measurement algorithm, and the like.

At the time of measurement, a user himself/herself wears the device 1 by wrapping the band 10 around the measurement site, and then starts measurement by pressing a button of the control body 12 or the like. At this time, if there is a physical connection failure such as the electrode 11 being lifted from the surface of the skin due to poor wrapping or tightening of the band 10, correct measurement cannot be performed. Therefore, in such a case, it is necessary to notify the user so that a measure such as re-attaching the band 10 can be taken. However, even when the physical connection between the electrodes 11 and the surface of the skin is good, the electrical connection between the electrodes 11 and the surface of the skin may be poor due to insufficient wetting. Therefore, simply evaluating whether the electrical connection between the electrodes 11 and the surface of the skin is good or poor does not allow distinguishing between a physical connection failure and insufficient wetting, and does not allow providing the user with appropriate guidance.

Therefore, the biological signal measurement device 1 determines whether the attachment state of the electrodes 11 is good or poor based on a feature amount extracted from a waveform of the biological signal measured during a period after an elapsed time from the attachment of the electrodes 11 reaches a predetermined time Tw. That is, whether the attachment state is good or poor is determined not immediately after the band 10 is attached, but after intentionally waiting for the predetermined time Tw. By providing such a waiting time, wetting between the electrodes 11 and the surface of the skin is expected to occur due to perspiration, and thus it becomes possible to determine whether there is a physical connection failure (that is, whether it is necessary to re-attach the electrodes 11) with high accuracy.

The predetermined time (waiting time) Tw may be set to a time of 5 minutes or more and 30 minutes or less. This is because, when such a time period elapses in a state in which the electrodes 11 are attached, a sufficient wetting state (that is, a state in which electrical connection between the electrodes 11 and the surface of the skin is maintained to such an extent that enables measurement of the biological signal and evaluation of the waveform are possible) is achieved for most users.

Hereinafter, as an embodiment of the present invention, a specific configuration example in a case where the present invention is applied to an upper arm electrocardiographic device will be described.

### <First Embodiment>

### (Device Configuration)

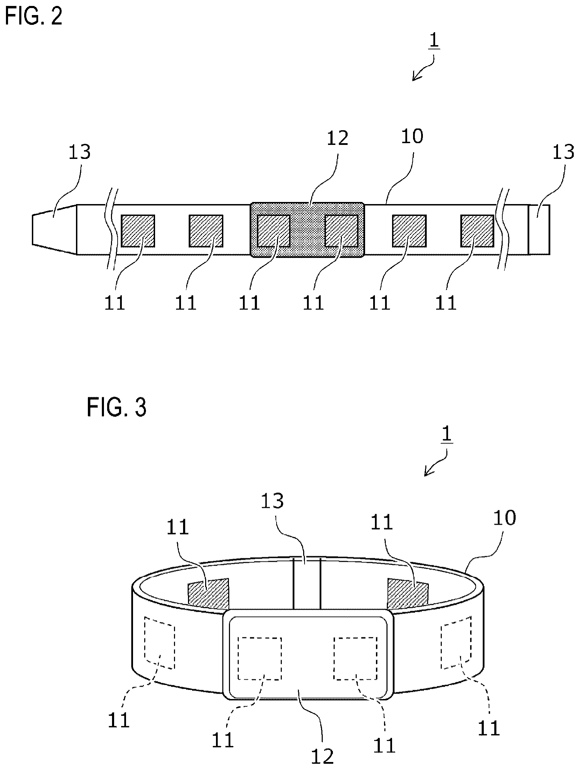

An embodiment of the present invention will be described with reference to FIGS. 1 to 3. FIG. 1 is a diagram illustrating a state in which the biological signal measurement device 1 is worn on an upper arm, FIG. 2 is a plan view of the biological signal measurement device 1, and FIG. 3 is a perspective view of the biological signal measurement device 1.

The biological signal measurement device 1 of the present embodiment is an upper arm electrocardiographic device that is worn on an upper arm (preferably, a left upper arm close to the heart) of a user and is used to measure an electrocardiogram (ECG) signal as a biological signal.

The biological signal measurement device 1 includes, as main components, a band 10, a plurality of electrodes 11 fixed to the band 10, and a control body 12 fixed to the band 10.

The band 10 is a member (fixing member) for fixing the electrodes 11 in a state in which the electrodes are pressed against the living body. In the present embodiment, a belt-shaped band 10 made of a flexible and pliable material (for example, chemical fiber, silicon, leather, or the like) is used. A fixing mechanism 13 is provided at a longitudinal end portion of the band 10. As illustrated in FIGS. 1 and 3, the biological signal measurement device 1 can be worn on the upper arm by forming the band 10 into a loop shape and fastening the band with the fixing mechanism 13. The fixing mechanism 13 may be any mechanism such as a hook-and-loop fastener, a hook, a connector, a button, or a magnet.

The plurality of electrodes 11 (also referred to as an electrode array) are embedded and fixed in the band 10 such that a contact surface with the living body is exposed to an inner side (living body side) of the band 10. The plurality of electrodes 11 are arranged in a line at equal intervals in the longitudinal direction of the band 10. Thus, when the band 10 is wrapped around the arm, the electrodes 11 come into contact with different circumferential positions of the arm. The number of electrodes 11 is not particularly limited and can be appropriately designed. At least two electrodes 11 (a pair of electrodes) may be provided to measure an ECG signal, and three or more electrodes 11 may be provided to increase the reliability and robustness of measurement. In the present embodiment, a configuration in which six electrodes 11 (three electrode pairs) are provided is adopted.

The electrode 11 is a dry-type metal electrode. The wet-type electrode (gel electrode or the like) has problems such as a possibility of causing skin rash or itching when attached for a long time, and low durability and maintainability, whereas the dry-type electrode 11 does not have such problems. The biological signal measurement device 1 of the present embodiment is assumed to be worn continuously for a long time and to monitor the ECG signal for 24 hours, and thus the electrodes 11 are preferably dry-type electrodes.

The control body 12 is a processing unit that performs control and signal processing of the biological signal measurement device 1. The control body 12 has a structure in which a processor, a memory, a battery, and other circuits are mounted inside a case made of resin or metal, for example. The control body 12 may be provided with a physical switch and a display. Although not illustrated, the control body 12 and the plurality of electrodes 11 are connected via signal lines.

### (Control Body)

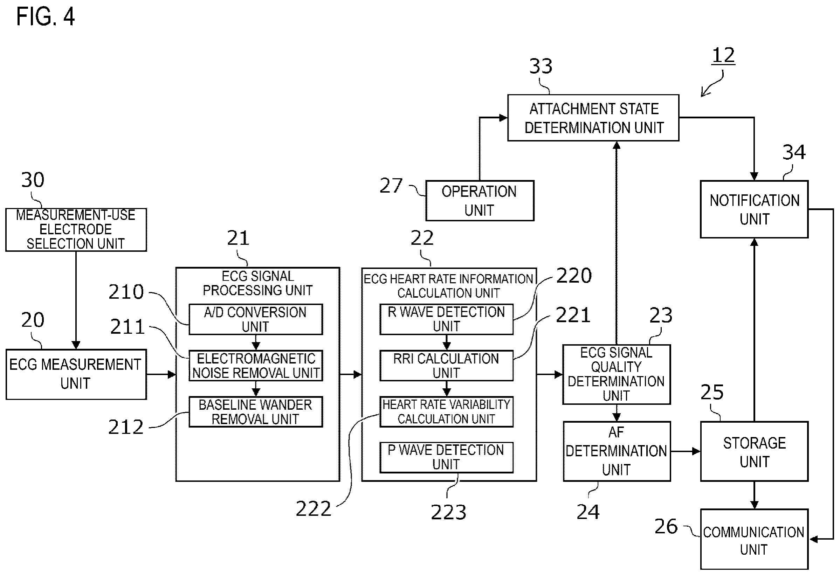

FIG. 4 is a block diagram illustrating an example of a functional configuration of the control body 12.

The control body 12 includes, as a configuration related to the measurement of the ECG signal, a measurement-use electrode selection unit 30, an ECG measurement unit 20, an ECG signal processing unit 21, an ECG heart rate information calculation unit 22, an ECG signal quality determination unit 23, an AF determination unit 24, a storage unit 25, a communication unit 26, and an operation unit 27. The measurement-use electrode selection unit 30 selects an electrode pair to be used for measurement of an ECG signal. The ECG measurement unit 20 is a circuit that amplifies a potential difference between the selected electrode pair by a differential amplifier and outputs the amplified potential difference as an ECG signal. The ECG signal processing unit 21 is a part that performs AD conversion and filtering processing of the ECG signal, and includes an AD conversion unit 210 that performs AD conversion of the ECG signal, an electromagnetic noise removal unit 211 that removes electromagnetic noise from the ECG signal to increase an SN ratio, and a baseline wander removal unit 212 that removes baseline wander (low-frequency wander) of the ECG signal. The ECG heart rate information calculation unit 22 is a part that extracts various types of heart rate information from the ECG signal, and includes an R wave detection unit 220, an RRI calculation unit 221, a heart rate variability calculation unit 222, and a P wave detection unit 223. The ECG signal quality determination unit 23 is a part that determines whether the quality of the ECG signal is good or poor (that is, whether accurate or reliable data suitable for diagnosis or the like can be measured). The ECG signal quality determination unit 23 further has a function as a waveform feature extraction unit that extracts a feature amount from a waveform of the ECG signal, and the extracted feature amount of the waveform is used for an attachment state determination process described below. The AF determination unit 24 is a part that detects an occurrence of AF (Atrial Fibrillation) based on the heart rate information and calculates an indicator related to AF. The storage unit 25 is a nonvolatile memory that stores measured or calculated data. The communication unit 26 is a part that performs wireless data communication with an external device (for example, a smartphone of a user, another health device, a home server, or the like). The operation unit 27 is an input interface for a user to perform an input operation. The operation unit 27 may be a physical button or a touch panel display.

In addition, the control body 12 includes an attachment state determination unit 33 and a notification unit 34, as components related to automatic determination of the attachment state of the electrodes 11. The attachment state determination unit 33 is a part that determines whether the attachment state of the electrodes 11 is good or poor based on the feature amount of the waveform of the ECG signal extracted by the ECG signal quality determination unit 23. The notification unit 34 is a part that performs notification to the user in accordance with a determination result of the attachment state determination unit 33.

### (ECG Signal Measurement Process)

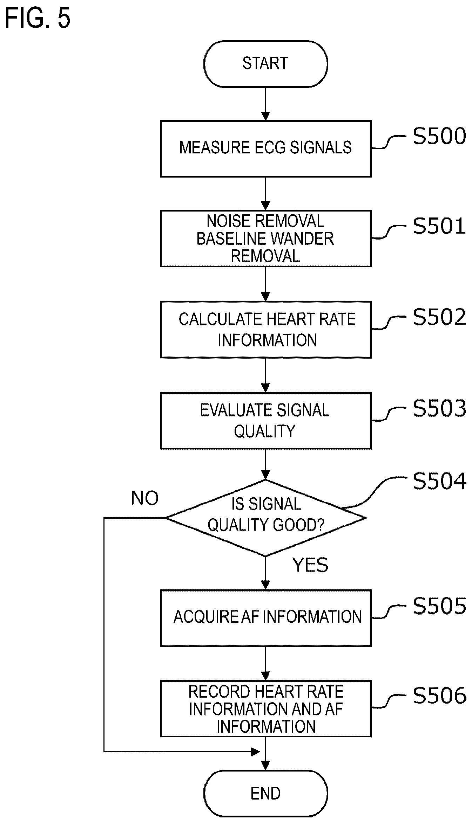

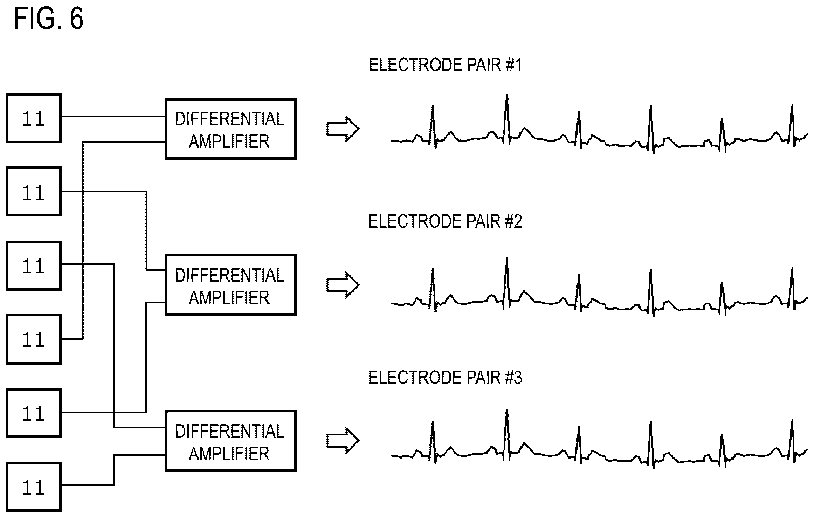

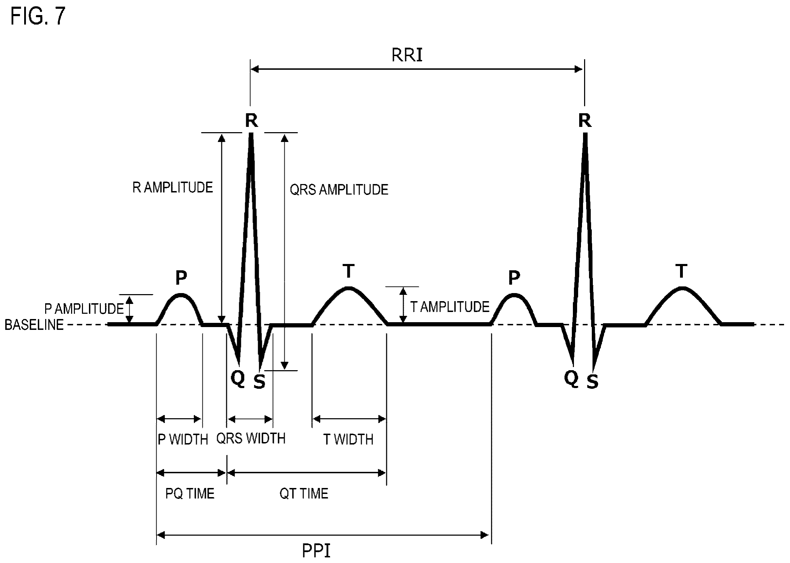

An ECG signal measurement process by the biological signal measurement device 1 will be described with reference to FIGS. 5 to 7. FIG. 5 is a flowchart illustrating a flow of an ECG signal measurement process, FIG. 6 is a diagram illustrating an example of an electrode pair and an ECG signal, and FIG. 7 is a diagram illustrating a waveform of an ECG signal and heart rate information.

When the user wraps the band 10 around the upper arm, fastens the band 10 with the fixing mechanism 13, and then performs an operation of instructing the start of measurement with the operation unit 27, the processor of the control body 12 starts the measurement process of FIG. 5.

In step S500, the ECG measurement unit 20 measures ECG signals of three channels using three electrode pairs. Specifically, the electrode pair (two electrodes 11) to be used for measurement is selected by the measurement-use electrode selection unit 30, and the potential difference between the selected electrode pair is amplified by the differential amplifier and taken in as an ECG signal (analog voltage signal). By sequentially switching the electrode pairs to be selected, ECG signals of three channels are taken in.

In the present embodiment, as illustrated in FIG. 6, three electrode pairs are set such that two electrodes 11 that are just opposite to each other when the band 10 is wrapped around the upper arm are paired. This is because a larger potential difference (i.e., an ECG signal having a higher SN ratio) can be measured when the two electrodes 11 forming a pair are separated from each other. However, the method of setting the electrode pairs is not limited thereto. For example, a multiplexer may be used to freely switch the combination of the electrodes 11 to be paired. In this case, ECG signals of four or more channels can be measured from the six electrodes 11.

In step S501, the ECG signal processing unit 21 performs AD conversion of the ECG signal, and removes electromagnetic noise and baseline wander by digital signal processing. A known noise reduction method such as a band-pass filter, a notch filter, or a moving average can be used to remove electromagnetic noise and baseline wander.

In step S502, the ECG heart rate information calculation unit 22 analyzes the taken-in ECG signal and calculates various types of heart rate information. The ECG heart rate information calculation unit 22 performs the processing of step S502 when time-series data (waveform data) of an ECG signal for a predetermined unit time is taken in from the ECG signal processing unit 21. The unit time may be set to, for example, a time of about 10 seconds to 600 seconds, and is set to 60 seconds in the present embodiment.

As schematically illustrated in FIG. 7, the waveform of one heart rate of the ECG signal mainly includes a P wave, a QRS wave, and a T wave. Note that the QRS wave indicates a waveform obtained by combining an R wave, which is an upward peak, and a downward Q wave and a downward S wave that appear before and after the R wave. In a normal heart, an electrical stimulus generated at the sinoatrial node is transmitted from the atrium to the ventricle, and excitation (contraction) of the atrium and excitation (contraction) of the ventricle occur in sequence, thereby pumping blood. The P wave is a waveform corresponding to the excitation of the atrium, the QRS wave is a waveform corresponding to the excitation of the ventricle, and the T wave is a waveform corresponding to the process of recovery of the ventricle from the excitation. In the case of an average adult, the heart rate is about 60 to 80 bpm, and thus, about 60 to 80 heart rate waveforms are included in the time-series data of the ECG signal for a unit time (60 seconds).

Examples of the heart rate information include an R amplitude (height of R wave from the baseline), a QRS amplitude (defined by, for example, the sum of the height of R wave from the baseline and the depth of S wave from the baseline), a P amplitude (height of P wave from the baseline), a T amplitude (height of T wave from the baseline), a P width (time from start of P wave to end of P wave), a QRS width (time from start of Q wave to end of S wave), a T width (time from start of T wave to end of T wave), a PQ time (time from start of P wave to start of Q wave), a QT time (time from start of Q wave to end of T wave), an RRI (RR interval; time from peak of R wave to peak of next R wave), a PPI (PP interval; time from start of P wave to start of next P wave), a heart rate variability (temporal variation in RRI and PPI), and the like. It is not necessary to calculate all of the heart rate information, and only necessary heart rate information may be calculated. In addition, other heart rate information may be calculated.

In step S503, the ECG signal quality determination unit 23 evaluates the quality of the ECG signal for the unit time taken-in in step S501. Any indicator may be used for the evaluation of the signal quality. For example, noise, power (intensity), baseline wander, or the like of the ECG signal itself may be used as the evaluation indicator, or the heart rate information calculated in step S502 (including whether the heart rate information has been successfully calculated) may be used as the evaluation indicator. When it is determined that the ECG signal for the unit time measured most recently does not satisfy the predetermined quality (accurate or reliable data suitable for diagnosis or the like) (NO in step S504), the ECG signal for the unit time and the heart rate information are discarded.

When the ECG signal of the predetermined quality is obtained (YES in step S504), the AF determination unit 24 detects the occurrence of AF (atrial fibrillation) based on the heart rate information, and calculates an indicator related to AF (step S505). The heart rate information calculated in step S502 and the AF information obtained in step S505 are recorded in the storage unit 25 together with the information on the measurement time (step S506).

The measurement process of FIG. 5 described above is repeatedly executed every unit time, whereby the ECG signal is monitored for 24 hours.

### (Attachment State Determination Process)

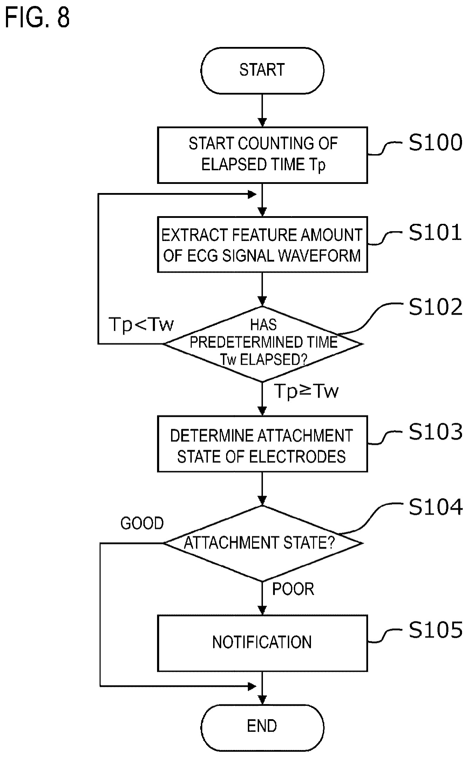

FIG. 8 illustrates a flow of an attachment state determination process in a first embodiment.

When the user wraps the band 10 around the upper arm, fastens the band 10 with the fixing mechanism 13, and then performs an operation of instructing the start of measurement with the operation unit 27, the processor of the control body 12 starts the measurement process of FIG. 5, and also starts the measurement process of FIG. 8.

When the attachment state determination unit 33 of the control body 12 detects that the user performs an operation of instructing the start of measurement, the attachment state determination unit 33 determines that the user has attached the electrodes 11, and starts counting of the "elapsed time Tp from the attachment of the electrodes 11" (step S100). Note that, in the present embodiment, the user operation is used as a trigger for counting the elapsed time Tp, but the counting of the elapsed time Tp may be started by detecting the attachment of the band 10 or the electrodes 11 to the arm by an optical/electrical/magnetic/physical sensor.

In step S101, the ECG signal quality determination unit 23 extracts a feature amount used for the attachment state determination process from the waveform of the ECG signal for the unit time. In the present embodiment, a section in which a state in which the ECG signal is saturated or 0 continues is referred to as a "non-signal section", and a length of the non-signal section is regarded as a feature amount.

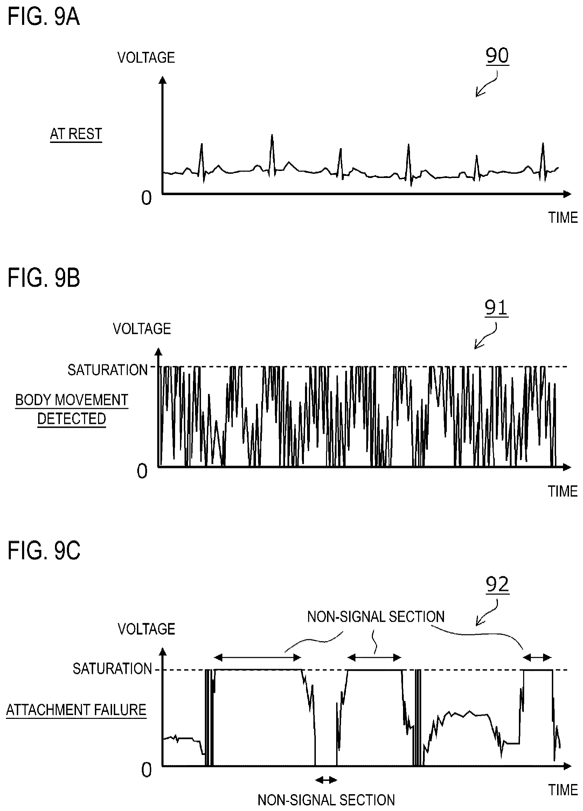

The non-signal section will be described in detail with reference to FIGS. 9A, 9B, and 9C. FIG. 9A illustrates an example of a waveform 90 of an ECG signal measured in a resting state, FIG. 9B illustrates an example of a waveform 91 of an ECG signal distorted by a body movement, and FIG. 9C illustrates an example of a waveform 92 of an ECG signal measured when there is an attachment failure of the electrodes 11. When the electrodes 11 separate from the surface of the skin, the differential voltage of the electrode pair cannot be correctly measured, and the ECG signal is saturated or becomes 0. Therefore, when the attachment state of the electrodes 11 is poor (physical connection is unstable), the state in which the ECG signal is saturated or 0 tends to appear continuously for a certain period of time, as illustrated by the waveform 92. As illustrated by the waveform 91, when myoelectric noise caused by the body movement is mixed in, the differential voltage is also distorted, and the ECG signal may be instantaneously saturated or swing to 0, but the state of saturation or 0 does not continue. Therefore, by evaluating the length of the non-signal section, it is possible to distinguish between the attachment failure of the electrodes 11 and the influence of the body movement.

The ECG signal quality determination unit 23 temporarily stores the feature amounts extracted from the ECG signals of the three electrode pairs in the storage unit 25. Note that when a plurality of non-signal sections appear in a waveform for a unit time, the longest time (the longest time of the non-signal section) may be adopted as the feature amount.

In step S102, the attachment state determination unit 33 determines whether the ECG signal used for extracting the feature amount in step S101 is a signal measured during a period after the elapsed time Tp reaches the predetermined time Tw. When the elapsed time Tp has not reached the predetermined time Tw (Tp < Tw), the process returns to step S101. In the present embodiment, for example, Tw is set to 10 minutes.

After the elapsed time Tp reaches the predetermined time Tw (Tp ≥ Tw), the attachment state determination unit 33 determines whether the attachment state of the electrodes 11 is good or poor based on the feature amount extracted from the waveform of the ECG signal (step S103). Specifically, when a non-signal section having a length equal to or greater than a predetermined value Tth appears in the waveform of the ECG signal of at least one of the electrode pairs, the attachment state determination unit 33 determines that there is a high possibility that electrode lift-off has occurred, and determines the attachment state to be "poor". When no non-signal section having a length equal to or greater than the predetermined value Tth is detected in the ECG signals of all the electrode pairs, the attachment state is determined to be "good". In the present embodiment, for example, Tth is set to 1 second.

When the attachment state is determined to be poor (step S104), the notification unit 34 notifies the user and prompts the user to re-wrap the band 10 (step S105). The method of notification is not particularly limited. For example, it is conceivable to issue a warning sound, to output a voice message prompting the user to re-wrap the band 10, to display a message on a display, to perform notification by vibration or light, to transfer a message to an external device (such as a smartphone possessed by the user), or the like.

In the determination process of the present embodiment, the contact state between the electrodes 11 and the surface of the skin is evaluated based on the ECG signal waveform not immediately after the attachment of the electrodes 11 but after a predetermined time has elapsed. By waiting until the wetting between the electrodes 11 and the surface of the skin is sufficiently increased by perspiration and then measuring and evaluating the ECG signal, a measurement failure due to insufficient wetting can be excluded as much as possible. Accordingly, a physical connection failure such as lift-off of the electrode 11 (that is, a state in which the band 10 needs to be re-wrapped) can be determined with high accuracy.

### (Other Feature Amounts)

In the above embodiment, the length of the non-signal section is extracted as the feature amount of the ECG signal waveform. However, the feature amount used for the determination as to whether the attachment state is good or poor may be other feature amounts. Hereinafter, as other examples of the feature amount, (1) a peak amplitude and (2) a degree of noise contamination will be described.

### (1) Peak Amplitude

Even when the physical connection state between the electrodes 11 and the surface of the skin is good and the ECG signal can be measured, if the intensity of the ECG signal is too weak and the peaks of the R wave, the P wave, the T wave, and the like are unclear, the accuracy and reliability of the heart rate information may be reduced, or the acquisition of the heart rate information may fail, which is not preferable. In such a case, there is a possibility of improvement by shifting the attachment positions of the electrodes 11 upward (to positions close to the shoulder). This is because the intensity of the ECG signal becomes stronger as the position is closer to the heart.

Therefore, for example, in step S101, the ECG signal quality determination unit 23 extracts the peak amplitude of the R wave of the ECG signal as the feature amount. Specifically, the value of the R amplitude or the QRS amplitude illustrated in FIG. 7 is used for the attachment state determination process. Note that, although several tens of R waves are included in the ECG signal waveform for a unit time, a representative value such as an average value, a maximum value, or a minimum value of the amplitude may be used as the feature amount. Then, in step S103, the attachment state determination unit 33 compares a peak amplitude A of the ECG signal with a predetermined amplitude value Ath, and determines the attachment state to be "poor" when the peak amplitude A is smaller than the predetermined amplitude value Ath (A < Ath).

The amplitude value Ath serving as a determination criterion may be a predetermined fixed value or may be dynamically determined from the measured ECG signal. The present inventors have found from the results of the subject experiment that the heart rate information can be acquired with high accuracy when the peak of the R wave exceeds three times the amplitude of the baseline undulation including the P wave and the T wave. Therefore, in the present embodiment, the effective value (root mean square) of the entire ECG signal is regarded as a value corresponding to the amplitude of the baseline undulation, and the determination reference amplitude value Ath is determined by the following formula.$Ath = 3 \times RMSecg$ Here, RMSecg is an effective value of the entire ECG signal or a partial section thereof.

### (2) Degree of Noise Contamination

Even when the physical connection state between the electrodes 11 and the surface of the skin is good and the ECG signal can be measured, if the wetting between the electrodes 11 and the surface of the skin is not sufficient yet, a lot of noise is mixed in the measured ECG signal, which may cause a decrease in the accuracy or reliability of the heart rate information or may cause a failure in acquiring the heart rate information. In such a case, it is desirable to resolve the lack of wetting by applying cream or lotion to the skin or moistening the electrodes 11.

Therefore, for example, in step S101, the ECG signal quality determination unit 23 extracts a degree of noise contamination in the ECG signal as the feature amount. In a waveform in which noise caused by insufficient wetting is mixed, a fluctuation of around 1 Hz appears in the baseline portion of the ECG signal. Therefore, for example, the effective value RMSecg of the entire ECG signal or a partial section thereof is calculated, and this value is used as an indicator ND of the degree of noise contamination. In step S103, the attachment state determination unit 33 compares the degree of noise contamination ND obtained from the ECG signal with a predetermined threshold value NDth, and determines the attachment state to be "poor" when the degree of noise contamination ND is equal to or greater than the threshold value NDth (ND ≥ NDth).

The threshold value NDth serving as a determination criterion may be a predetermined fixed value or may be dynamically determined from the measured ECG signal. The present inventors have found from the results of the subject experiment that the effective value RMSecg is equal to or greater than the R amplitude in the waveform in which the noise caused by the insufficient wetting is mixed. Therefore, the threshold value NDth may be set to be approximately the same as, for example, the R amplitude or the QRS amplitude.

### <Second Embodiment>

It is considered that, in most cases, the insufficient wetting can be resolved by providing the waiting time of the predetermined time Tw, as in the first embodiment. However, there may be a certain number of individuals for whom sufficient wetting between the electrodes 11 and the skin is not achieved even after a sufficient time has elapsed. It may also depend on the environment (temperature, humidity, etc.) at the site. If the cause is the insufficient wetting, the situation cannot be improved by re-wrapping the band 10, and it becomes necessary to take a measure to increase the wetting between the electrodes 11 and the surface of the skin, such as applying cream or lotion to the skin or moistening the electrode. In addition, as described above, when the amplitude of the peak is small, it is effective to bring the attachment positions of the electrodes 11 close to the heart. Therefore, in a second embodiment, a cause of the poor attachment state is determined based on the feature amount extracted from the waveform of the ECG signal, and the notification to the user is varied in accordance with the cause of the poor attachment state.

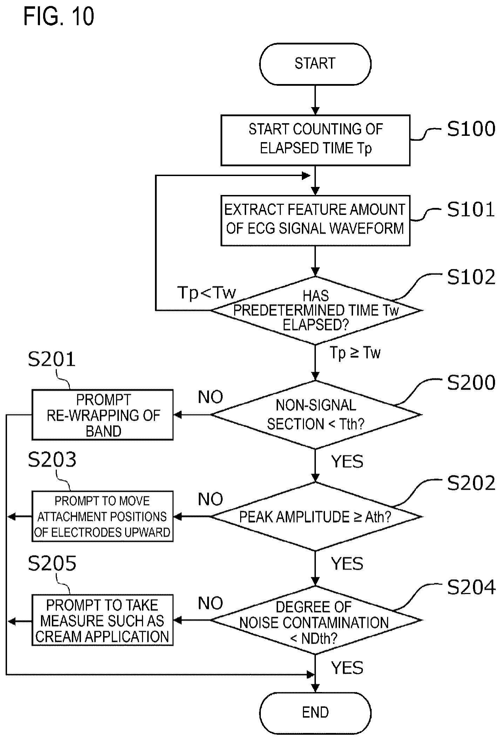

FIG. 10 illustrates a flow of an attachment state determination process in the second embodiment. The same step numbers are given to the same parts as those in the determination process of the first embodiment. Hereinafter, the processing different from that of the first embodiment will be mainly described.

In step S101, the ECG signal quality determination unit 23 extracts three types of feature amounts, namely, "length of non-signal section", "peak amplitude", and "degree of noise contamination", from the ECG signal waveform for the unit time. The definition and extraction method of each feature amount are the same as those in the first embodiment.

After the elapsed time Tp reaches the predetermined time Tw (step S102), the attachment state determination unit 33 inspects whether the length of the non-signal section is smaller than the predetermined value Tth (step S200), whether the peak amplitude is equal to or greater than the predetermined amplitude value Ath (step S202), and whether the degree of noise contamination is smaller than the predetermined threshold value NDth (step S204), respectively. When the determination of YES is obtained in all the inspections, the attachment state is determined to be "good".

If there is a non-signal section having a length equal to or greater than the predetermined value Tth (NO in step S200), it is considered that the cause of the attachment failure is a physical connection failure such as lift-off of the electrode, and the notification unit 34 prompts the user to re-wrap the band 10 (step S201). If the peak amplitude is smaller than the predetermined amplitude value Ath (NO in step S202), it is considered that the cause of the attachment failure is that the attachment positions of the electrodes 11 are far from the heart, and the notification unit 34 prompts the user to move the attachment positions of the electrodes 11 upward (to positions close to the shoulder) (step S203). If the degree of noise contamination is equal to or greater than the predetermined threshold value NDth (NO in step S204), it is considered that the cause of the attachment failure is insufficient wetting between the electrodes 11 and the surface of the skin, and the notification unit 34 prompts the user to take a measure to increase the wetting, such as applying cream (step S205).

According to the determination process of the present embodiment, in addition to the same operational effects as those of the first embodiment, there is an advantage in that it is possible to provide a measurement device with excellent usability, which can guide the user to take an appropriate measure when insufficient wetting is not resolved due to the user's constitution or the environment, when the peak of the ECG signal is small, or the like.

### <Third Embodiment>

In the first and second embodiments, the determination of the attachment state of the electrodes 11 is performed after the predetermined time Tw has elapsed. However, when the attachment failure can be clearly determined before the predetermined time Tw has elapsed, the user may be notified at that time.

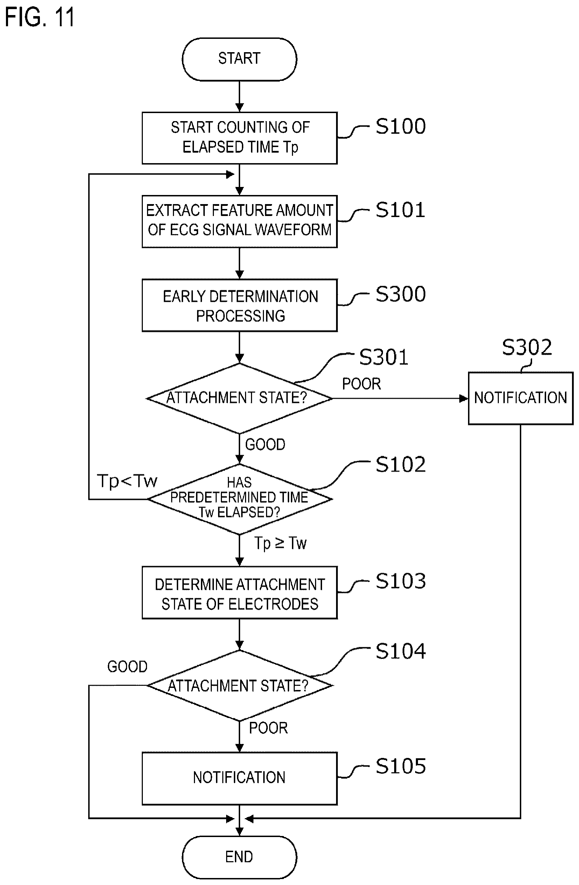

FIG. 11 illustrates a flow of an attachment state determination process in a third embodiment. The same step numbers are given to the same parts as those in the determination process of the first embodiment. Hereinafter, the processing different from that of the first embodiment will be mainly described.

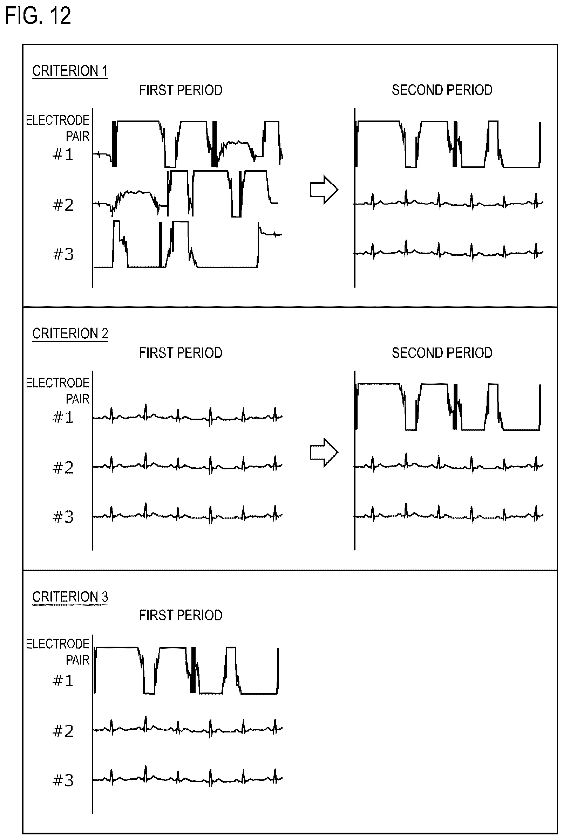

After the feature amount of the ECG signal waveform is extracted in step S101, the attachment state determination unit 33 determines whether the attachment state of the electrodes 11 is good or poor based on the feature amount extracted from immediately after the attachment of the electrodes 11 to the present time in step S300. The determination processing of the step S300 is referred to as "early determination processing" in order to distinguish it from the determination processing of the step S103. When the attachment failure is determined in the early determination processing (step S301), the notification is immediately performed and the process is terminated (step S302). In the early determination processing, it is evaluated whether the feature amount (the value of the feature amount or the temporal change thereof) extracted from the waveform of the ECG signal measured during a period before the elapsed time Tp reaches the predetermined time Tw satisfies a predetermined criterion. FIG. 12 illustrates an example of the predetermined criterion.
(1) The criterion 1 is such that the non-signal section having a length equal to or greater than the predetermined value Tth appears in all of the three electrode pairs in a first period, but the non-signal section having the length equal to or greater than the predetermined value Tth does not appear in some of the electrode pairs in a subsequent second period.
   When the skin is dry, there is a possibility that the electrical connection between the electrodes 11 and the skin may not be properly established immediately after the attachment of the electrodes 11. In such a case, initially, the non-signal section appears in all the electrode pairs. When wetting progresses due to perspiration and the electrical connection becomes good, the non-signal section usually disappears in all the electrode pairs. However, if the non-signal section disappears in some electrode pairs but the non-signal section still appears in the remaining electrode pairs, there is a high probability that a physical connection failure such as electrode lift-off has occurred in the remaining electrode pairs. Therefore, when the criterion 1 is satisfied, the attachment state determination unit 33 may immediately determine the attachment failure, and the notification unit 34 may prompt the user to re-wrap the band 10.
(2) The criterion 2 is such that the non-signal section having a length equal to or greater than the predetermined value Tth does not appear in all of the three electrode pairs in the first period, but the non-signal section having the length equal to or greater than the predetermined value Tth appears in some of the electrode pairs in the subsequent second period.
   When the electrodes 11 are securely in contact with and fixed to the surface of the skin, a state without the non-signal section does not change to a state with the non-signal section. In other words, when a change from a state without the non-signal section to a state with the non-signal section occurs as in the criterion 2, the physical connection state between the electrodes 11 and the surface of the skin is unstable, and there is a high probability that the electrode is in contact or separated due to the posture or the body movement of the user. Therefore, in this case, the attachment state determination unit 33 may immediately determine the attachment failure, and the notification unit 34 may prompt the user to re-wrap the band 10.
(3) The criterion 3 is such that an electrode pair in which the non-signal section having a length equal to or greater than the predetermined value Tth appears and an electrode pair in which the non-signal section having the length equal to or greater than the predetermined value Tth does not appear are mixed in the three electrode pairs.

Since the wetting states of the six electrodes 11 are substantially the same, it is normal that the non-signal section appears in all of the three electrode pairs or the non-signal section does not appear in all of the three electrode pairs. In other words, when the electrode pair having the non-signal section and the electrode pair having no non-signal section are mixed as in the criterion 3, there is a high probability that the connection failure such as electrode lift-off has occurred in the electrode pair having the non-signal section. Therefore, in this case, the attachment state determination unit 33 may immediately determine the attachment failure, and the notification unit 34 may prompt the user to re-wrap the band 10. Since the criterion 3 can be determined only by the ECG signal waveform for one period, the determination can be made even immediately after the attachment of the electrodes 11.

By combining the above-described determination processing, when the attachment failure can be clearly determined, it is possible to determine the attachment failure before the elapsed time Tp reaches the predetermined time Tw and to prompt the user to take a measure. Therefore, according to the present embodiment, the convenience of the device can be further enhanced. Note that, although the example in which the early determination processing is incorporated into the determination flow of the first embodiment has been described here, the early determination processing may be incorporated into the determination flow of the second embodiment.

### <Others>

The embodiments described above are merely illustrative of configuration examples of the present invention. The present invention is not limited to the specific aspects described above, and various modifications are possible within the scope of the technical idea of the present invention. For example, the body part on which the device is worn may be other than the upper arm. In addition, as the fixing member for fixing the electrodes 11 in a state in which the electrodes are pressed against the living body, a band-shaped (belt-shaped) member that is wrapped around the measurement portion of the living body in a state in which the electrodes 11 are brought into contact, as in the above embodiments, an envelope-shaped member that covers and wraps the measurement portion of the living body, or a ring-shaped member may be used. Additionally, the fixing member may have elasticity or deformability in order to correspond to the size (diameter) of the measurement portion of the living body. Alternatively, when the fixing member is made of a non-elastic material, the fixing member may have a structure that allows adjustment of the length. The number of electrodes 11 may be one or more. The arrangement of the plurality of electrodes 11 is not necessarily one line, and the electrodes 11 may be arranged in a two-dimensional array. In addition, the electrodes 11 may be arranged at irregular intervals, instead of at equal intervals. The electrode 11 may be integrated with the fixing member (band 10), or may have a separate structure from the fixing member. The control body 12 need not be fixed to the band 10. For example, the control body 12 and the band 10 may be formed as separate structures, and the control body 12 and the band 10 (the electrode 11) may be connected to each other by a cable. The biological signal to be measured is not limited to the ECG signal, and may be, for example, a myoelectric signal or a brain wave signal.

The present specification includes the following disclosures.

### [Supplementary Note 1]

A biological signal measurement device (1) including:
a dry-type electrode(11);
a member (10) configured to fix the electrode (11) in a state in which the electrode (11) is pressed against a living body; and
a control body (12) configured to measure a biological signal by the electrode (11), in which
the control body (12) includes:
   a waveform feature extraction unit (23) configured to extract a feature amount from a waveform of the biological signal, and
   an attachment state determination unit (33) configured to determine whether an attachment state of the electrode (11) is good or poor, based on a feature amount extracted from a waveform of a biological signal measured during a period after an elapsed time from attachment of the electrode (11) to the living body by the member (10) reaches a predetermined time.

### [Supplementary Note 2]

The biological signal measurement device (1) according to Supplementary Note 1, in which the predetermined time is set to a time of 5 minutes or more.

### [Supplementary Note 3]

The biological signal measurement device (1) according to Supplementary Note 1 or 2, in which
the waveform feature extraction unit (23) extracts, as the feature amount, a length of a non-signal section in which a state in which the biological signal is saturated or 0 continues, and
the attachment state determination unit (33) determines the attachment state to be poor when the non-signal section having a length equal to or greater than a predetermined value appears.

### [Supplementary Note 4]

The biological signal measurement device (1) according to any one of Supplementary Notes 1 to 3, in which
the attachment state determination unit (33) determines the attachment state to be poor before the elapsed time reaches the predetermined time, when a feature amount extracted from a waveform of a biological signal measured during a period before the elapsed time reaches the predetermined time satisfies a predetermined criterion.

### [Supplementary Note 5]

The biological signal measurement device (1) according to Supplementary Note 4, in which
the dry-type electrode includes a plurality of electrode pairs (11, 11),
the waveform feature extraction unit (23) extracts, as the feature amount, a length of a non-signal section in which a state in which the biological signal is saturated or 0 continues, and
the predetermined criterion is a fact that the non-signal section having a length equal to or greater than a predetermined value appears in all of the plurality of electrode pairs (11, 11) in a first period, but the non-signal section having the length equal to or greater than the predetermined value does not appear in some of the plurality of electrode pairs (11, 11) in a second period after the first period.

### [Supplementary Note 6]

The biological signal measurement device (1) according to Supplementary Note 4 or 5, in which
the dry-type electrode includes a plurality of electrode pairs (11, 11),
the waveform feature extraction unit (23) extracts, as the feature amount, a length of a non-signal section in which a state in which the biological signal is saturated or 0 continues, and
the predetermined criterion is a fact that the non-signal section having a length equal to or greater than a predetermined value appears in none of the plurality of electrode pairs (11, 11) in a first period, but the non-signal section having the length equal to or greater than the predetermined value appears in some of the plurality of electrode pairs (11, 11) in a second period after the first period.

### [Supplementary Note 7]

The biological signal measurement device (1) according to any one of Supplementary Notes 4 to 6, in which
the dry-type electrode includes a plurality of electrode pairs (11, 11),
the waveform feature extraction unit (23) extracts, as the feature amount, a length of a non-signal section in which a state in which the biological signal is saturated or 0 continues, and
the predetermined criterion is a fact that an electrode pair (11, 11) in which the non-signal section having a length equal to or greater than a predetermined value appears and an electrode pair (11, 11) in which the non-signal section having the length equal to or greater than the predetermined value does not appear are mixed in the plurality of electrode pairs (11, 11).

### [Supplementary Note 8]

The biological signal measurement device according to any one of Supplementary Notes 1 to 7, further including a notification unit (34) configured to notify a user when the attachment state determination unit (33) determines the attachment state to be poor.

### [Supplementary Note 9]

The biological signal measurement device (1) according to Supplementary Note 8, in which
the attachment state determination unit (33) determines a cause of a poor attachment state, based on a feature amount extracted from a waveform of the biological signal, and
the notification unit (34) varies a notification to the user in accordance with the cause of the poor attachment state.

### [Supplementary Note 10]

The biological signal measurement device (1) according to Supplementary Note 8 or 9, in which
the waveform feature extraction unit (23) extracts, as the feature amount, a length of a non-signal section in which a state in which the biological signal is saturated or 0 continues, and
the notification unit (34) prompts the user to re-attach the electrode (11) when the attachment state determination unit (33) determines that the non-signal section having a length equal to or greater than a predetermined value appears.

### [Supplementary Note 11]

The biological signal measurement device (1) according to any one of Supplementary Notes 8 to 10, in which
the waveform feature extraction unit (23) extracts, as the feature amount, an amplitude of a peak included in the biological signal, and
the notification unit (34) prompts the user to change an attachment position of the electrode (11) when the attachment state determination unit (33) determines that the amplitude of the peak is smaller than a predetermined amplitude value.

### [Supplementary Note 12]

The biological signal measurement device (1) according to any one of Supplementary Notes 8 to 11, in which
the waveform feature extraction unit (23) extracts, as the feature amount, a degree of noise contamination in the biological signal, and
the notification unit (34) prompts the user to take a measure to increase wetting between the electrode (11) and a surface of a skin when the attachment state determination unit (33) determines that the degree of noise contamination is equal to or greater than a predetermined threshold value.

### [Supplementary Note 13]

The biological signal measurement device (1) according to any one of Supplementary Notes 1 to 12, in which
the control body (12)
includes an operation unit (27) that is operated by a user after the user attaches the electrode (11), and
starts counting of the elapsed time when the user operates the operation unit (27).

### [Supplementary Note 14]

The biological signal measurement device (1) according to any one of Supplementary Notes 1 to 13, in which the biological signal is an electrocardiogram (ECG) signal.

### [Supplementary Note 15]

The biological signal measurement device according to any one of Supplementary Notes 1 to 14, in which the member (10) is a band (10) provided with the electrode (11).

### [Supplementary Note 16]

The biological signal measurement device (1) according to Supplementary Note 15, in which the band (10) is attached to an upper arm of the living body.

### [Supplementary Note 17]

A method for controlling a biological signal measurement device (1) configured to measure a biological signal by a dry-type electrode (11), the method including: counting an elapsed time from attachment of the electrode (11) to a living body; extracting a feature amount from a waveform of the biological signal; and determining whether an attachment state of the electrode (11) is good or poor, based on a feature amount extracted from a waveform of a biological signal measured during a period after the elapsed time reaches a predetermined time.

### REFERENCE SIGNS

1: Biological signal measurement device
10: Band
11: Electrode
12: Control body
13: Fixing mechanism

## Claims

1. A biological signal measurement device comprising:
a dry-type electrode;
a member configured to fix the electrode in a state in which the electrode is pressed against a living body; and
a control body configured to measure a biological signal by the electrode,
wherein
the control body includes:
a waveform feature extraction unit configured to extract a feature amount from a waveform of the biological signal, and
an attachment state determination unit configured to determine whether an attachment state of the electrode is good or poor, based on a feature amount extracted from a waveform of a biological signal measured during a period after an elapsed time from attachment of the electrode to the living body by the member reaches a predetermined time.

2. The biological signal measurement device according to claim 1, wherein the predetermined time is set to a time of 5 minutes or more.

3. The biological signal measurement device according to claim 1 or 2, wherein
the waveform feature extraction unit extracts, as the feature amount, a length of a non-signal section in which a state in which the biological signal is saturated or 0 continues, and
the attachment state determination unit determines the attachment state to be poor when the non-signal section having a length equal to or greater than a predetermined value appears.

4. The biological signal measurement device according to any one of claims 1 to 3, wherein
the attachment state determination unit determines the attachment state to be poor before the elapsed time reaches the predetermined time, when a feature amount extracted from a waveform of a biological signal measured during a period before the elapsed time reaches the predetermined time satisfies a predetermined criterion.

5. The biological signal measurement device according to claim 4, wherein
the dry-type electrode includes a plurality of electrode pairs,
the waveform feature extraction unit extracts, as the feature amount, a length of a non-signal section in which a state in which the biological signal is saturated or 0 continues, and
the predetermined criterion is a fact that the non-signal section having a length equal to or greater than a predetermined value appears in all of the plurality of electrode pairs in a first period, but the non-signal section having the length equal to or greater than the predetermined value does not appear in some of the plurality of electrode pairs in a second period after the first period.

6. The biological signal measurement device according to claim 4 or 5, wherein
the dry-type electrode includes a plurality of electrode pairs,
the waveform feature extraction unit extracts, as the feature amount, a length of a non-signal section in which a state in which the biological signal is saturated or 0 continues, and
the predetermined criterion is a fact that the non-signal section having a length equal to or greater than a predetermined value appears in none of the plurality of electrode pairs in a first period, but the non-signal section having the length equal to or greater than the predetermined value appears in some of the plurality of electrode pairs in a second period after the first period.

7. The biological signal measurement device according to any one of claims 4 to 6, wherein
the dry-type electrode includes a plurality of electrode pairs,
the waveform feature extraction unit extracts, as the feature amount, a length of a non-signal section in which a state in which the biological signal is saturated or 0 continues, and
the predetermined criterion is a fact that an electrode pair in which the non-signal section having a length equal to or greater than a predetermined value appears and an electrode pair in which the non-signal section having the length equal to or greater than the predetermined value does not appear are mixed in the plurality of electrode pairs.

8. The biological signal measurement device according to any one of claims 1 to 7, further comprising a notification unit configured to notify a user when the attachment state determination unit determines the attachment state to be poor.

9. The biological signal measurement device according to claim 8, wherein
the attachment state determination unit determines a cause of a poor attachment state, based on a feature amount extracted from a waveform of the biological signal, and
the notification unit varies a notification to the user in accordance with the cause of the poor attachment state.

10. The biological signal measurement device according to claim 8 or 9, wherein
the waveform feature extraction unit extracts, as the feature amount, a length of a non-signal section in which a state in which the biological signal is saturated or 0 continues, and
the notification unit prompts the user to re-attach the electrode when the attachment state determination unit determines that the non-signal section having a length equal to or greater than a predetermined value appears.

11. The biological signal measurement device according to any one of claims 8 to 10, wherein
the waveform feature extraction unit extracts, as the feature amount, an amplitude of a peak included in the biological signal, and
the notification unit prompts the user to change an attachment position of the electrode when the attachment state determination unit determines that the amplitude of the peak is smaller than a predetermined amplitude value.

12. The biological signal measurement device according to any one of claims 8 to 11, wherein
the waveform feature extraction unit extracts, as the feature amount, a degree of noise contamination in the biological signal, and
the notification unit prompts the user to take a measure to increase wetting between the electrode and a surface of a skin when the attachment state determination unit determines that the degree of noise contamination is equal to or greater than a predetermined threshold value.

13. The biological signal measurement device according to any one of claims 1 to 12, wherein
the control body
includes an operation unit that is operated by a user after the user attaches the electrode, and
starts counting of the elapsed time when the user operates the operation unit.

14. The biological signal measurement device according to any one of claims 1 to 13, wherein the biological signal is an electrocardiogram (ECG) signal.

15. The biological signal measurement device according to any one of claims 1 to 14, wherein the member is a band provided with the electrode.

16. The biological signal measurement device according to claim 15, wherein the band is attached to an upper arm of the living body.

17. A method for controlling a biological signal measurement device configured to measure a biological signal by a dry-type electrode, the method comprising:
counting an elapsed time from attachment of the electrode to a living body;
extracting a feature amount from a waveform of the biological signal; and
determining whether an attachment state of the electrode is good or poor, based on a feature amount extracted from a waveform of a biological signal measured during a period after the elapsed time reaches a predetermined time.
